(19) Europäisches Patentamt / European Patent Office / Office européen des brevets

(11) EP 1 887 024 B1

(12) # EUROPÄISCHE PATENTSCHRIFT

(45) Veröffentlichungstag und Bekanntmachung des Hinweises auf die Patenterteilung:
**14.10.2009 Patentblatt 2009/42**

(51) Int Cl.:
***C08G 77/388*** *(2006.01)* ***D06M 15/643*** *(2006.01)*
***A61K 8/898*** *(2006.01)*

(21) Anmeldenummer: **07111703.0**

(22) Anmeldetag: **04.07.2007**

(54) **Neuartige Polysiloxane mit quaternären Ammoniumgruppen, Verfahren zu deren Herstellung und deren Verwendung in reinigenden und pflegenden Formulierungen**

New polysiloxanes with quaternary ammonium groups, method for their manufacture and their use in cleaning and treating formulations

Polysiloxane d'un nouveau type doté de groupes d'ammonium quaternaires, son procédé de fabrication et son utilisation dans des formulation de nettoyage et de soin

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **31.07.2006 DE 102006035512**

(43) Veröffentlichungstag der Anmeldung:
**13.02.2008 Patentblatt 2008/07**

(73) Patentinhaber: **Evonik Goldschmidt GmbH**
**45127 Essen (DE)**

(72) Erfinder:
- **Herrwerth, Sascha Dr.**
**45134, Essen (DE)**
- **Ferenz, Michael Dr.**
**45147, Essen (DE)**
- **Leidreiter, Holger Dr.**
**45529, Hattingen (DE)**
- **Müller, Felix Dr.**
**42555, Velbert (DE)**
- **Peggau, Jörg**
**45357, Essen (DE)**

(56) Entgegenhaltungen:
**EP-A- 0 017 122**
**DE-A1- 1 493 384**
**US-A- 4 895 964**
**US-A- 5 446 185**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

**Beschreibung**

Die Erfindung betrifft neuartige Polysiloxane mit quaternären Ammoniumgruppen sowie ein Verfahren zu deren Herstellung. Sie betrifft ferner die Verwendung dieser Polymeren als Additiv in Formulierungen für die Pflege und Reinigung von Haut und Hautanhangsgebilden, wie beispielsweise als Konditioniermittel für Haare, sowie zur Reinigung und Pflege harter Oberflächen, wie als Additiv in der Fahrzeugwäsche.

Polysiloxane mit quaternären Gruppen und deren Anwendung als Additive für Haarpflege oder Textilweichmacher sind aus der Patentliteratur bekannt. So werden zum Beispiel in der DE AS 14 93 384 Strukturen beschrieben, bei denen Siloxane seitenständig mit statistisch über das Polymer verteilten Ammoniumgruppen modifiziert sind. Diese Verbindungen haben den Nachteil, dass ein ausgeprägter Siliconcharakter vermindert wird und eine gute Wirksamkeit nicht mehr zu beobachten ist.

Einen ausgeprägteren Siliconecharakter haben kationische Silicone, wie sie in DE 37 19 086 und EP 0 282 720 beschrieben werden. Die EP 0 282 720 beschreibt Strukturen, bei denen die quaternären Funktionen endständig an das Siloxan gebunden sind. Derartige Verbindungen bieten Vorteile hinsichtlich ihrer Wirkung als Konditioniermittel sowohl für Haare und Textilien als auch für harte Oberflächen. Hier allerdings stehen nur zwei kationische Zentren zur Verfügung, was oftmals nur zu einer unzureichenden Substantivität auf der Oberfläche führt.

Aus der DE-OS 33 40 708 sind polyquaternäre Polysiloxan-Polymere bekannt. Polyquaternäre Polysiloxan-Polymere dieses Typs weisen die oben beschriebenen Nachteile nicht auf. Der praktischen Verwendung dieser Verbindungen steht jedoch deren aufwändiges Herstellverfahren entgegen. Die Verbindungen sind in wirtschaftlich nicht vertretbaren Ausbeuten von < 60 % der Theorie herstellbar.

Menschliches Haar ist täglich den verschiedensten Einflüssen ausgesetzt. Neben mechanischen Beanspruchungen durch Bürsten, Kämmen, Hochstecken oder Zurückbinden werden die Haare auch durch Umwelteinflüsse wie zum Beispiel starke UV-Strahlung, Kälte, Wind und Wasser angegriffen. Auch der physiologische Status (z.B. Alter, Gesundheit) der jeweiligen Person beeinflusst den Zustand der keratinischen Fasern.

Insbesondere die Behandlung mit chemischen Mitteln verändert Struktur und Oberflächeneigenschaften der Haare. Methoden wie zum Beispiel das Dauerwellen, Bleichen, Färben, Tönen, Glätten usw., aber auch häufiges Waschen mit aggressiven Tensiden tragen dazu bei, dass mehr oder weniger starke Schäden an der Haarstruktur verursacht werden. So wird zum Beispiel bei einer Dauerwelle sowohl der Cortex als auch die Cuticula des Haares angegriffen. Die DisulfidBrücken des Cystins werden durch den Reduktionsschritt aufgebrochen und im anschließenden Oxidationsschritt zum Teil zu Cysteinsäure oxidiert.

Beim Bleichen wird nicht nur das Melanin zerstört, sondern es werden außerdem ca. 15 bis 25 % der Disulfid-Bindungen des Cystins bei einer milden Bleiche oxidiert. Bei einer exzessiven Bleichung können es sogar bis zu 45 % sein (K. F. de Polo, A Short Textbook of Cosmetology, 2000, Verlag für chemische Industrie, H. Ziolkowsky GmbH).

So ergeben sich aus den chemischen Behandlungen, dem häufigen Waschen oder der UV-Bestrahlung nachteilige mechanische Eigenschaften für das Haar, hervorgerufen durch Entfernung natürlich ausgeschiedener Haarfette bzw. -feuchthaltemittel (Sebum). Es wird dadurch spröde, trocken, glanzlos, porös und schlecht kämmbar.

Außerdem ist gründlich gereinigtes Haar für gewöhnlich sehr schwer zu kämmen, sowohl in nassem als auch in trockenem Zustand, da die einzelnen Haare dazu neigen, kraus zu werden und sich zu verknoten. So verliert es erst beim Waschen und anschließend beim Kämmen seine Widerstandsfähigkeit. Dies zeigt sich in einer signifikanten Abnahme der Zug-Dehnungsfestigkeit bei nassem Haar. Außerdem ist es gegenüber einer weiteren Schädigung durch Chemikalien, Tenside und Umwelteinflüsse weniger widerstandsfähig als gesundes Haar.

Für die Pflege derart geschädigter Haare gibt es spezielle Zubereitungen, wie zum Beispiel Haarspülungen, Haarkuren, Shampoos, Leave-in Konditionierer usw., die jedoch vor allem die Kämmbarkeit, den Griff und den Glanz geschädigter Haare verbessern können. Derartige handelsübliche Haarpflegemittel enthalten hauptsächlich kationische Tenside auf Alkylammonium-Basis, Polymere, Wachse bzw. Öle oder Siliconöle. Die Wirksamkeit dieser Verbindungen lässt sich u.a. auf eine Hydrophobierung der Haar-Oberfläche zurückführen.

Bei allen diesen Mitteln wird zwar eine gute Pflegewirkung (Konditionierung) der Haare erreicht, aber das Aussehen, insbesondere der Glanz der Haare, wird durch die Pflegeprodukte nicht verbessert, sondern teilweise sogar verschlechtert.

Es besteht daher also weiterhin ein Bedarf an vielseitig einsetzbaren Wirkstoffen für Körperreinigungs- und Pflegemittel wie Shampoos, Haarbehandlungsmittel und Haarnachbehandlungsmittel, die neben der reinigenden Wirkung die Pflege des Haares verbessern und gleichzeitig guten Glanz verleihen, die das Haar vor der Schädigung der Haarstruktur schützen und die bereits verursachten strukturellen Schädigungen des Haares, hervorgerufen durch Umwelteinflüsse sowie form- und farbgebende Behandlungen, minimieren.

Es ist eine Aufgabe der Erfindung, einen solchen Wirkstoff zur Verfügung zu stellen, der in der Lage ist, sowohl Eigenschaften wie Kämmbarkeit, Weichheit, Volumen, Formbarkeit, Handhabbarkeit, die Entwirrbarkeit von ungeschädigten und geschädigten Haaren zu verbessern, als auch dem Haar einen schönen Glanz zu verleihen. Die Verbindungen sollen also eine verbesserte oder zumindest gleich gute Einzelwirkung, insgesamt aber eine verbesserte kombinierte

Wirkung von mechanischen und anderen Eigenschaften zeigen.

Die Reinigung und Pflege harter Oberflächen im privaten und gewerblichen Bereich erfordert zum Teil komplexe Formulierungen und vorgegebene, geregelte Arbeitsabläufe. So besteht zum Beispiel das Waschen von Fahrzeugen in Autowaschanlagen in der Regel aus mehreren nacheinander ablaufenden Vorgängen, die genau aufeinander abgestimmt werden müssen. Diese Abstimmung umfasst u.a. die richtige Wahl der chemischen Formulierungen, die Einhaltung der Einwirkungszeiten, die Mechanik der Reinigung und die Temperaturwahl. Weiterführende Literatur: F. Müller, J. Peggau, S. Arif, Special Purpose Cleaning Formulations: Auto Care, in Handbook of Detergents, Part D: Formulation, M. Showell, ed. CRC Press, Boca Raton 2006, pp. 261 bis 278.

Die eigentliche Reinigung, die in eine Vor- und Hauptwäsche unterteilt sein kann, wobei jeweils verschiedene Grundformulierungen eingesetzt werden können, beinhaltet die Entfernung der festen, unlöslichen Schmutzpartikel auf der Fahrzeugoberfläche. Dafür gibt es eine große Anzahl an Formulierungen für die unterschiedlichsten Reinigungsverfahren. Diese Formulierungen bestehen normalerweise aus anionischen Tensidsystemen, die zusammen mit basischen oder sauren Komponenten die für die Reinigung notwendige Tensioaktivität einbringen.

An diese Reinigung schließt sich der Spülvorgang an, bei dem Reinigungsmittelreste entfernt werden müssen. Dieser Schritt dient der Vorbereitung für die Anwendung eines geeigneten Trocknungsmittels, das vor der abschließenden Gebläsetrocknung das Fahrzeug hydrophobiert und so der verbleibende Wasserfilm leichter entfernt werden kann. Das Spülen ist deshalb wichtig, da Trocknungsmittel einen kationischen Charakter haben und sich sonst nach der Anwendung anionischer Reinigungsformulierungen schwerlösliche Salze bilden würden, die auf dem Fahrzeug zu unansehnlichen Flecken führen und so weder zum gewünschten Glanzeffekt noch zur Hydrophobierung führen.

Kationische Tenside bilden bei Anwendungen, wo Substantivität gefordert ist, d.h. ein Verbleiben der oberflächenaktiven Verbindung auf dem behandelten Gut, die wesentlichen Inhaltsstoffe dieser Formulierungen. Wie bei Anwendungen im Bereich Weichspüler, Textilausrüstung oder Haarspülungen so findet diese Stoffklasse auch bei Trockneranwendungen in Autowaschanlagen ihre Verbreitung.

Da auch Fahrzeuglacke, wie die meisten Oberflächen, ein negatives elektrisches Potential aufweisen, breiten sich nach dem Aufsprühen der Trocknungsmittelformulierung die kationischen Tenside auf dem Fahrzeug aus und verdrängen den vorhandenen Wasserfilm. Dieser Vorgang, der als "Aufriss" bezeichnet wird, resultiert in einer Assoziation des Wasserfilmes zu Tropfen. Diese Tropfen laufen dann sowohl durch die eigene Schwerkraft als auch durch Benutzung eines Gebläses im letzten Schritt der Autoreinigung vom Fahrzeug herunter.

Die Formulierung von Trocknungshilfen für die automatische Fahrzeugreinigung stellt den Formulierer vor besondere Aufgaben. So muss die Formulierung nicht nur einen spontanen Wasseraufriss erzeugen, sie soll auch zu einer schnellen Trocknung und einem langanhaltenden Glanz führen. Wichtig ist dabei die richtige Anwendungskonzentration, die bei etwa 0,1 bis 0,3 % liegen sollte. Ist die Konzentration zu niedrig, so wird der Wasserfilm nicht aufreißen, ist sie zu hoch, so bildet sich auf der Fahrzeugoberfläche eine schmierige, fettige Schicht, die nicht mehr zum gewünschten Glanzeffekt führen kann.

Die Formulierung soll auch bei tiefen Temperaturen klar und ohne Ausfällungen bleiben. Darüber hinaus muss das Produkt eine große Wasserhärtetoleranz besitzen, um sowohl im harten und weichem als auch im wiederaufbereitetem Wasser nicht zu Trübungen zu führen. Eventuell verwendete Wachse, Öle oder andere, nicht mit Wasser mischbare Pflegemittel, die auf der Oberfläche bleiben sollen, müssen emulgiert werden.

Eine Grundformulierung für einen Trockner besteht in der Regel aus quaternären Ammoniumverbindungen, sogenannten Quats. Dabei kommen heute fast ausschließlich umweltfreundliche Esterquats oder Imidazolinquats zur Anwendung, bei denen die Fettkette hauptsächlich aus Ölsäure besteht. Da Quats meistens nicht wasserlöslich sind, erleichtern diese hochungesättigten Fettketten die Formulierung in wässrigen Systemen.

Neben Quats sind noch Rohstoffe mit emulgierenden Eigenschaften notwendig, um das o.a. Anforderungsprofil zu gewährleisten.

Im Zuge der Beschleunigung des Betriebs von Autowaschstrassen sind diverse Versuche unternommen worden, den relativ langwierigen Aufrissprozess zu beschleunigen. Es wurden zum Beispiel Siliconverbindungen, wie in der DE 101 07 772 beschrieben, getestet, jedoch ohne Erfolg. Da einer der geschwindigkeitsbestimmenden Schritte in der Autowaschstrasse der Ablauf des Trockners ist, wird eine Beschleunigung den Durchsatz an Fahrzeugen in der Waschstrasse erhöhen und damit Wartezeiten für Kunden reduzieren und die Anlageneffizienz erhöhen.

Dieser Erfindung liegt die Aufgabe zugrunde, quaternäre Polysiloxan-Polymere zu finden, welche sich mit guten Ausbeuten herstellen lassen und darüber hinaus in den jeweiligen Anwendungen das gewünschte Eigenschaftsprofil aufweisen und eine sehr gute Konditionierwirkung und Glanz auf Haaren zeigen.

Dieser Erfindung liegt des weiteren die Aufgabe zugrunde, quaternäre Polysiloxan-Polymere zu finden, welche sich mit guten Ausbeuten herstellen lassen und darüber hinaus in den jeweiligen Anwendungen das gewünschte Eigenschaftsprofil aufweisen sowie einen deutlich beschleunigtem Aufriss bei gleichzeitigem Glanzerhalt in der Autopflege besitzen.

Überraschenderweise wurde gefunden, dass Polysiloxane der allgemeinen Formel I $[M' D_n]_3 T$ dieses leisten.

Ein Gegenstand der Erfindung sind daher Polysiloxane der allgemeinen Formel I

$$[M' D_n]_3 T$$

Formel I

Dabei sind:

M' = $XSiY_2O_{1/2}$

D = $SiY_2O_{2/2}$

T = $SiZO_{3/2}$

X sind gleiche oder verschiedene organische Reste, die Ammoniumfunktionen tragen,

Y sind gleiche oder verschiedene Reste aus der Gruppe Alkyl, Aryl, oder Alkaryl mit 1 bis 30 C-Atomen, vorzugsweise Methyl oder Phenyl, insbesondere Methyl,

Z sind gleiche oder verschiedene Reste aus der Gruppe Alkyl, Aryl, oder Alkaryl mit 1 bis 30 C-Atomen,vorzugsweise Methyl oder Phenyl,

n ist 2 bis 200 , bevorzugt 3 bis 120, insbesondere 8 bis 80.

Geeignete Reste X sind zum Beispiel Gruppen mit dem Aufbau -R1-R2, worin

R1 vorzugsweise gleiche oder verschiedene zweiwertige Reste sind, ausgewählt aus der Gruppe

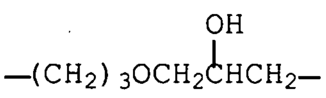
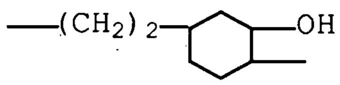

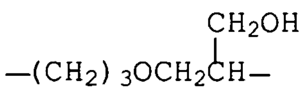
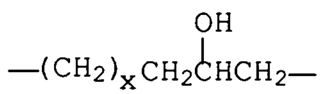

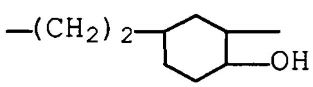
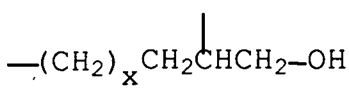

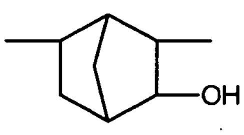
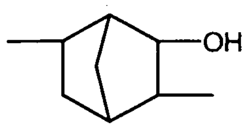

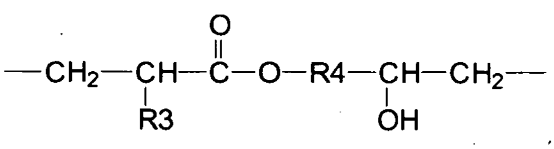
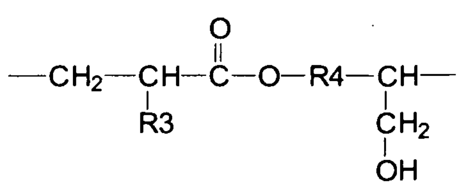

R1 ist bevorzugt:

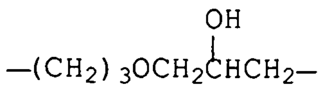

R2 ausgewählt ist aus der Gruppe bestehend aus

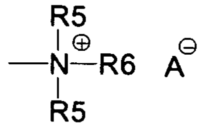
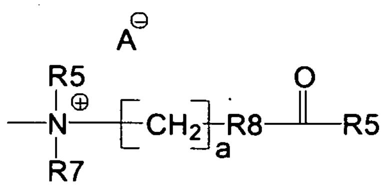

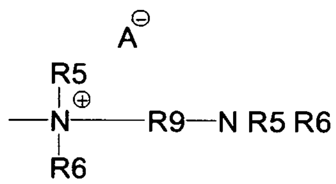
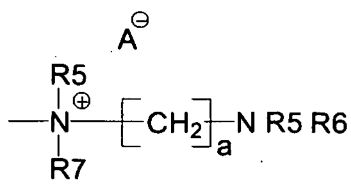

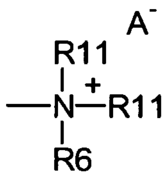
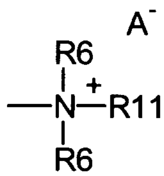

R3 sind gleiche oder verschiedene Reste aus der Gruppe Wasserstoff oder Alkyl mit 1 bis 6 C-Atomen, bevorzugt Methyl,

R4 sind gleiche oder verschiedene zweiwertige Kohlenwasserstoffreste, die gegebenenfalls Etherfunktionen enthalten, bevorzugt Methylen,

R5, R6, R7 sind jeweils unabhängig voneinander Wasserstoff oder Alkylreste mit 1 bis 30 C-Atomen,

R8 sind gleiche oder verschiedene Reste aus der Gruppe -O-; -NR10,

R9 sind gleiche oder verschiedene gegebenenfalls verzweigte divalente Kohlenwasserstoffreste,

R10 sind gleiche oder verschiedene Reste aus der Gruppe Wasserstoff oder Alkyl mit 1 bis 6 C-Atomen,

R11 sind gleiche oder verschiedene Reste der allgemeinen Formel:

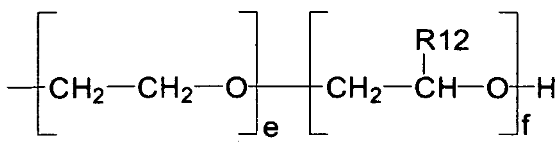

R12 sind gleiche oder verschiedene Alkyl-, Aryl- oder Alkarylreste mit 1 bis 30 C-Atomen, die gegebenenfalls Etherfunktionen enthalten, bevorzugt Methyl, Ethyl oder Phenyl, insbesondere Methyl,

e 0 bis 20, bevorzugt 0 bis 10, insbesondere 1 bis 3,

f 0 bis 20, bevorzugt 0 bis 10,

e + f >= 1,

x 2 bis 18,

a 2 bis 18, vorzugsweise 3,

$A^-$ sind gleiche oder verschiedene Gegenionen zu den positiven Ladungen an den quaternierten Stickstoffgruppen, ausgewählt aus anorganischen oder organischen Anionen der Säuren HA, sowie deren Derivate.

Es ist dem Fachmann geläufig, dass die Verbindungen in Form eines Gemisches mit einer im Wesentlichen durch statistische Gesetze geregelten Verteilung vorliegen.

Die Einheit T ist im statistischen Mittel einmal in einer Polymerkette vorhanden. Es liegt jedoch eine Mischung von Molekülen vor, so dass ein gewisser Anteil der Molekülen keine oder mehrere T-Einheiten besitzen.

In einer weiteren bevorzugten Ausführungsform der vorliegenden Erfindung besteht das Gegenion $A^-$ zu den positiven Ladungen an den quaternierten Stickstoffgruppen aus dem Anion einer physiologisch verträglichen Säure HA, die besonders bevorzugt aus Essigsäure, L-Hydroxycarbonsäure, insbesondere Milchsäure, oder aromatischen Carbonsäuren ausgewählt ist.

Weitere bevorzugte Gegenionen stammen aus gängigen Quaternierungsmitteln. Dies sind insbesondere Ethylsulfat, Methylsulfat, Toluolsulfonat, Chlorid und Bromid.

Ein weiterer Gegenstand dieser Erfindung ist ein Verfahren zur Herstellung der erfindungsgemäßen Produkte. Dieses geht von der Equlibrierung von Aryltris(dimethylsiloxy)silan und/oder Alkyltris(dimethylsiloxy)silan, insbesondere Phenyltris(dimethylsiloxy)silan und Methyltris(dimethylsiloxy)silan mit Octamethylcylotetrasiloxan und/oder Decamethylcyclopentasiloxan (Cyclen) aus. Geeignete Methoden zur Equilibrirung von Siloxanen werden zum Beispiel in der Patentschrift EP 1 439 200 beschrieben.

Neben cyclischen Siloxanen können bei der Equilibrierung auch $\alpha$-$\omega$-di-SiH-funktionelle Siloxane oder nicht funktionelle Siloxane der Equilibriermischung zugesetzt werden, um den mittleren Modifikationsgrad gezielt zu erniedrigen.

Mit den so erhaltenen SiH-funktionellen Siloxanen lassen sich anschließend Doppelbindungen enthaltende Epoxyde, wie zum Beispiel Allylglycidether, hydrosilylieren. Dem Fachmann ist bekannt, dass für die Hydrosilylierung Pt-, Rh-oder Ru-Katalysatoren verwendet werden.

Die so erhaltenen Epoxysiloxane können schließlich mit tertiären Aminen zu den gewünschten quaternäre Ammonium-funktionen tragenden Siloxanen umgesetzt werden.

Es ist dem Fachmann bekannt, dass im Rahmen einer derartigen Reaktionssequenz sowohl bei der Equilibrierung der SiH-funktionellen Siloxane, als auch bei der Hydrosilylierung und der Quaternisierung mit Nebenreaktionen zu rechnen ist. Der Umfang der Nebenreaktionen hängt unter anderem von der Art der Edukte als auch von den Reaktionsbedingungen ab. So liegt zum Beispiel der Quaternisierungsgrad bei der Umsetzung von Epoxysiloxanen mit tertiären Aminen in Gegenwart von Carbonsäuren nach gängigen Methoden bei etwa 80 bis 95 %.

Ein weiterer Gegenstand dieser Erfindung ist die Verwendung der Verbindungen der allgemeinen Formel I und der gemäß dem erfindungsgemäßen Verfahren hergestellten Verbindungen der allgemeinen Formel I bzw. der gemäß diesem Verfahren hergestellten technischen Mischungen als Additiv in gegebenenfalls tensidhaltigen wässrigen Pflege- und Reinigungsformulierungen.

Ein weiterer Gegenstand dieser Erfindung ist die Verwendung der Verbindungen der allgemeinen Formel I und der gemäß dem erfindungsgemäßen Verfahren hergestellten Verbindungen der allgemeinen Formel I bzw. der gemäß diesem Verfahren hergestellten technischen Mischungen als Konditioniermittel für Haarbehandlungsmittel und Haarnachbehandlungsmittel sowie als Mittel zur Verbesserung der Haarstruktur.

Ein weiterer Gegenstand dieser Erfindung ist die Verwendung der Verbindungen der allgemeinen Formel I und der gemäß dem erfindungsgemäßen Verfahren hergestellten Verbindungen der allgemeinen Formel I bzw. der gemäß diesem Verfahren hergestellten technischen Mischungen zur Herstellung von glanzverbessernden Pflegeformulierungen.

Ein weiterer Gegenstand dieser Erfindung sind Haarbehandlungsmittel und Haarnachbehandlungsmittel zum Ausspülen oder zum Verbleib im Haar, beispielsweise Shampoos mit oder ohne ausgeprägter Konditionierwirkung, 2inl-Shampoos, Spülungen, Haarkuren, Haarmasken, Frisierhilfen, Stylingmittel, Fönlotionen, Haarfestiger, Dauerwellmittel, Haarglättungsmittel und Mittel zum Färben der Haare. Je nach Anwendungszweck sind solche Mittel enthaltend 2 bis 25 Gew.- % eines oder mehrerer waschaktiver Tenside aus der Gruppe der anionischen, nichtionischen, amphoteren

oder zwitterionischen Tenside, 0,5 bis 10 Gew.-% eines oder mehrerer Emulgatoren, 0,5 bis 10 Gew.-% eines oder mehrerer Konsistenzgeber, 0,5 bis 10 Gew.-% eines oder mehrerer vorzugsweise kationischer Tenside oder Emulgatoren, 1 bis 20 Gew.-% eines oder mehrerer kosmetischer Öle, Siliconöle oder Emollients sowie übliche Hilfs- und Zusatzstoffe in üblichen Konzentrationen, und zusätzlich enthaltend ein oder mehrere haarkosmetische Wirkstoffe, ausgesucht aus der Gruppe der kationischen Polymere wie zum Beispiel quaternierte Cellulose und dessen Derivate, Chitosan und dessen Derivate, kationische Alkylglycoside, kationische Guar-Derivate, Polymere aus Dimethyldiallylammoniumsalzen und deren Copolymere mit Estern und Amiden von Acrylsäure und Methacrylsäure, Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere, Vinylpyrrolidon-Vinylimidazoliummethochlorid-Copolymere, Terpolymere aus den Monomeren Vinylpyrrolidon, Caprolactam und Acrylamiden, quaternierter Polyvinylalkohol sowie solche Polymere die unter den INCI-Bezeichnungen Polyquaternium-2, Polyquaternium-17, Polyquaternium-18, Polyquaternium-27 und Polyquaternium-37 bekannt sind, kationische oder nichtionische Proteinhydrolysate pflanzlichen oder tierischen Ursprungs auf Basis Keratin, Collagen, Elastin, Weizen, Reis, Soja, Milch, Seide, Mais oder weitere Siliconderivate, wie zum Beispiel Dimethiconol oder Dimethicone (INCI Bezeichnungen für Polydimethylsiloxane) sowie modifizierte Silicone, die terminal funktionalisiert (INCI Vorsilbe Bis-) und/oder propffunktionalisiert sein können, als da sind beispielsweise Alkoxysilicone und Alkylsilicone mit langkettigen Alkylgruppen, polyoxyalkylmodifizierte Silicone wie PEG/PPG-3/10 Dimethicone oder Bis-PEG/PPG-20/20 Dimethicone mit oder ohne Alkylethergruppe und deren Ester, wie zum Beispiel Dimethicone PEG-7 Cocoate sowie multifunktionalisierte Silicone wie zum Beispiel Cetyl PEG/PPG-10/1 Dimethicone oder Methyleugenyl PEG-8 Dimethicone, darüber hinaus Siliconcopolymere mit Acrylaten, einschließlich solcher Copolymere mit und ohne Alkylmodifizierung, verzweigte Siliconderivate wie Dimethicone/Silsesquioxane Copolymer, vernetzte Siliconcopolymere wie Dimethicone Crosspolymer, Alkyl Dimethicone/Divinyldimethicone Crosspolymer, Cetearyl Dimethicone Crosspolymer oder Cetearyl Dimethicone/Vinyl Dimethicone Crosspolymer, aminofunktionalisierte Silicone wie Amodimethicone, Aminopropyl Dimethicone, PEG-7 Amodimethicone, Methoxy PEG/PPG-7/3 Aminopropyl Dimethicone oder ionisch modifizierter Silicone wie Dimethicone Propyl PG-Betaine, Vitamine, Panthenol, Pyrrolidoncarbonsäure, Bisabolol, Pflanzenextrakte, Kreatin, Ceramide sowie UV-absorbierende Mittel, dadurch gekennzeichnet, dass sie eine wirksame Menge mindestens einer Verbindung der allgemeinen Formel (I) enthalten.

Ein weiterer Gegenstand dieser Erfindung ist die Verwendung der Verbindungen der allgemeinen Formel I und der gemäß dem erfindungsgemäßen Verfahren hergestellten Verbindungen der allgemeinen Formel I bzw. der gemäß diesem Verfahren hergestellten technischen Mischungen zur Herstellung von Formulierungen zur Reinigung und Pflege von harten Oberflächen, vorzugsweise zur Reinigung und Pflege von Fahrzeugen, insbesondere als Additiv in Trocknungshilfen für Autowaschstraßen.

Ausführungsbeispiele

Nachfolgende Beispiele sollen zur Verdeutlichung der Erfindung dienen, sie stellen jedoch keinerlei Einschränkung dar.

Beispiel 1:

a) Equilibrierung eines SiH-funktionellen Polysiloxans

In einem 500 mL Dreihalskolben wurden 8,2 g Phenyltris(dimethylsiloxy)silan, 334 g Decamethylpentasiloxan, 0,34 g eines sauren Katalysators gemischt und 4 Stunden bei 80 °C gerührt. Nach dem Abkühlen wurden 20 g $NaHCO_3$ zugegeben und bei Raumtemperatur 12 Stunden gerührt. Nach einer Filtration wurde ein klares Produkt mit einem SiH-Wert von 0,02 % erhalten.

b) Herstellung eines Epoxysiloxans

In einem 500 mL Dreihalskolben wurden 300 g der gemäß unter 1 a) hergestellten Verbindung und 7,5 g Allylglycidylether zusammen vorgelegt und auf 100 °C aufgeheizt. Anschließend wurden 15 ppm eines PlatinKatalysators zugesetzt und zwei Stunden lang gerührt. Nach einer anschließenden Reaktion erhielt man ein klares Produkt mit einem Epoxywert von 0,37 %.

c) Umsetzung zum quaternären Polysiloxan-Polymer

In einem 500 mL Dreihalskolben wurden 14,5 g 3-N,N-Dimethyl-aminopropyl-laurylsäureamid, 2,7 g Essigsäure und 120 g Isopropanol bei Raumtemperatur gerührt. Anschließend wurden 200 g der gemäß 1 b) hergestellten Verbindung

zugetropft. Anschließend wurde 8 Stunden bei 50 °C gerührt und destilliert. Es wurde eine trübe hochviskose Flüssigkeit erhalten, welches durch die folgende statistische Formel beschrieben wird:

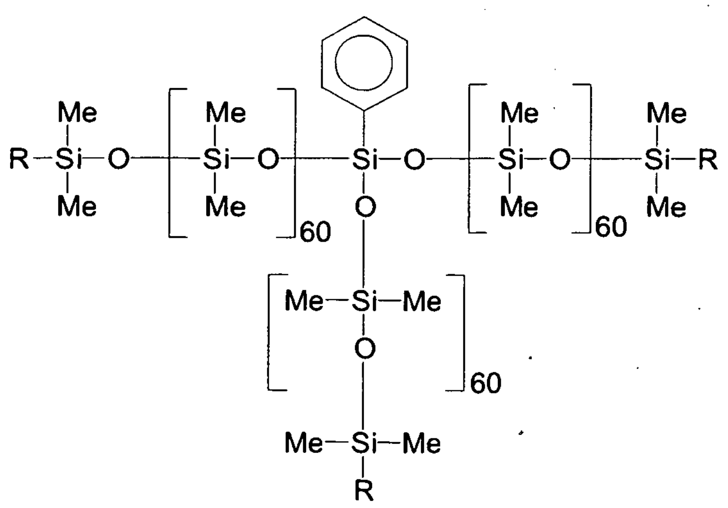

R =

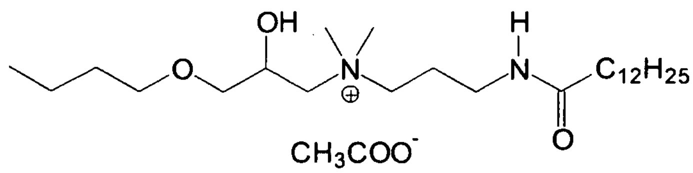

Dem Fachmann ist geläufig, dass die oben angegebene Formel eine idealisierte Strukturformel darstellt. Im Produkt liegen zusätzlich lineare und höher verzweigte Strukturen vor.

<u>Beispiel 2:</u>

a) Equilibrierung eines SiH-funktionellen Polysiloxans

In einem 1000 mL Dreihalskolben wurden 50 g Phenyltris(dimethylsiloxy)silan, 667 g Decamethylpentasiloxan, 0,7 g eines sauren Katalysators gemischt und 4 Stunden bei 80°C gerührt. Nach dem Abkühlen wurden 15 g $NaHCO_3$ zugegeben und bei Raumtemperatur 12 Stunden gerührt. Nach einer Filtration wurde ein klares Produkt mit einem SiH-Wert von 0,07 % erhalten.

b) Herstellung eines Epoxysiloxans

In einem 1000 mL Dreihalskolben wurden 670 g der gemäß unter 2 a) hergestellten Verbindung und 65 g Allylglycidylether zusammen vorgelegt und auf 100 °C aufgeheizt. Anschließend wurden 15 ppm eines Platin-Katalysators zugesetzt und zwei Stunden lang gerührt. Nach einer anschließenden Reaktion erhielt man ein klares Produkt mit einem Epoxywert von 0,99 %.

c) Umsetzung zum Siliconquat

In einem 1000 mL Dreihalskolben wurden 63 g N,N-Dimethyl-stearylamin, 12 g Essigsäure und 200 g Isopropanol bei Raumtemperatur gerührt. Anschließend wurden 325 g der gemäß 2 b) hergestellten Verbindung zugetropft. Anschließend wurde 8 Stunden bei 60 °C gerührt und destilliert. Es wurde eine trübe hochviskose Flüssigkeit erhalten, welches durch die folgende statistische Formel beschrieben wird:

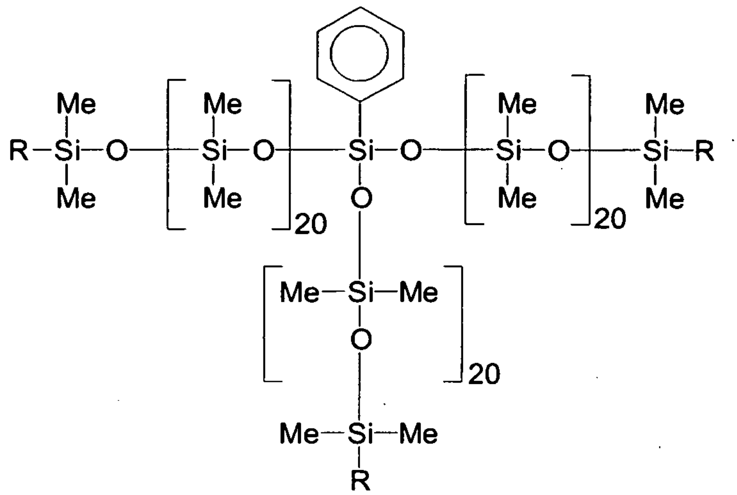

R =

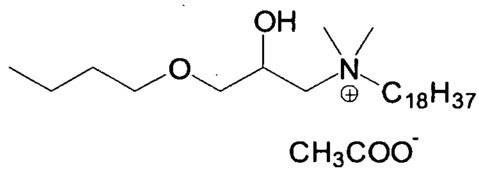

Dem Fachmann ist geläufig, dass die oben angegebene Formel eine idealisierte Strukturformel darstellt. Im Produkt liegen zusätzlich lineare und höher verzweigte Strukturen vor.

Beispiel 3:

a) Equilibrierung eines SiH-funktionellen Polysiloxans

In einem 500 mL Dreihalskolben wurden 33,1 g Phenyltris(dimethylsiloxy)silan, 274,4 g Decamethylpentasiloxan, 0,3 g eines sauren Katalysators gemischt und 4 Stunden bei 80 °C gerührt. Nach dem Abkühlen wurden 10 g $NaHCO_3$ zugegeben und bei Raumtemperatur 12 Stunden gerührt. Nach einer Filtration wurde ein klares Produkt mit einem SiH-Wert von 0,097 % erhalten.

b) Herstellung eines Epoxysiloxans

In einem 250 mL Dreihalskolben wurden 103,5 g der gemäß unter 3 a) hergestellten Verbindung und 15 g Allylglycidylether zusammen vorgelegt und auf 100 °C aufgeheizt. Anschließend wurden 15 ppm eines PlatinKatalysators zugesetzt und zwei Stunden lang gerührt. Nach einer anschließenden Reaktion erhielt man ein klares Produkt mit einem Epoxywert von 1,4 %.

c) Umsetzung zum Siliconquat

In einem 500 mL Dreihalskolben wurden 32 g 3-N,N-Dimethyl-aminopropyl-laurylsäureamid, 6,2 g Essigsäure und 100 g Isopropanol bei Raumtemperatur gerührt. Anschließend wurden 115 g der gemäß 3 b) hergestellten Verbindung zugetropft. Anschließend wurde 8 Stunden bei 60 °C gerührt und destilliert. Es wurde eine trübe hochviskose Flüssigkeit erhalten, welches durch die folgende statistische Formel beschrieben wird:

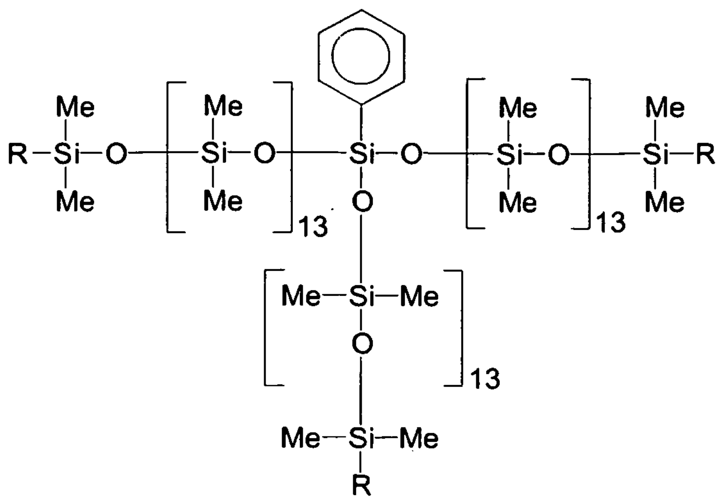

R = 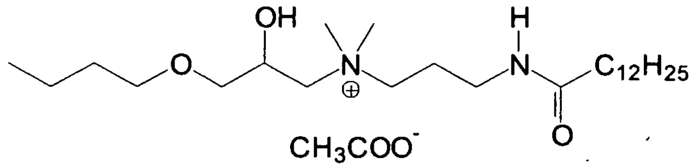

Dem Fachmann ist geläufig, dass die oben angegebene Formel eine idealisierte Strukturformel darstellt. Im Produkt liegen zusätzlich lineare und höher verzweigte Strukturen vor.

Beispiel 4:

a) Equilibrierung eines SiH-funktionellen Polysiloxans

In einem 500 mL Dreihalskolben wurden 3,3 g Phenyltris(dimethylsiloxy)silan, 220,4 g Decamethylpentasiloxan, 0,3 g eines sauren Katalysators gemischt und 4 Stunden bei 80 °C gerührt. Nach dem Abkühlen wurden 4,5 g $NaHCO_3$ zugegeben und bei Raumtemperatur 12 Stunden gerührt. Nach einer Filtration wurde ein klares Produkt mit einem SiH-Wert von 0,013 % erhalten.

b) Herstellung eines Epoxysiloxans

In einem 1000 mL Dreihalskolben wurden 752 g der gemäß unter 4 a) hergestellten Verbindung und 14,8 g Allylglycidylether zusammen vorgelegt und auf 100 °C aufgeheizt. Anschließend wurden 15 ppm eines PlatinKatalysators zugesetzt und zwei Stunden lang gerührt. Nach einer anschließenden Reaktion erhielt man ein klares Produkt mit einem Epoxywert von 0,21 %.

c) Umsetzung zum Siliconquat

In einem 250 mL Dreihalskolben wurden 3,2 g 3-N,N-Dimethyl-aminopropyl-laurylsäureamid, 0,62 g Essigsäure und 50 g Isopropanol bei Raumtemperatur gerührt. Anschließend wurden 76,2 g der gemäß 4 b) hergestellten Verbindung zugetropft. Anschließend wurde 8 Stunden bei 60 °C gerührt und destilliert. Es wurde eine trübe hochviskose Flüssigkeit erhalten, welches durch die folgende statistische Formel beschrieben wird:

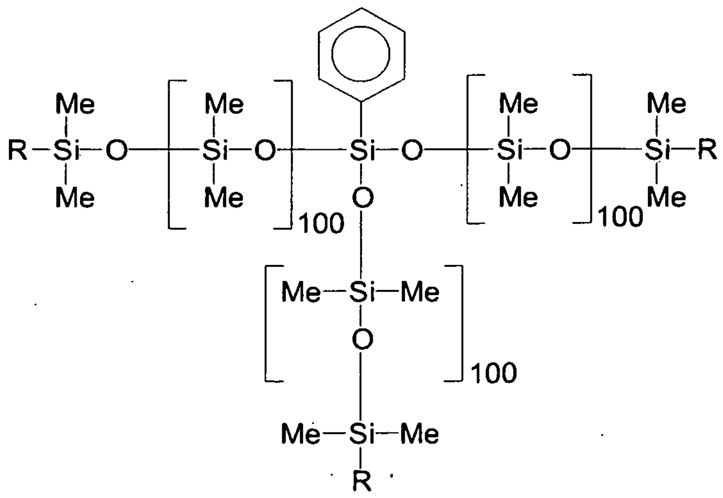

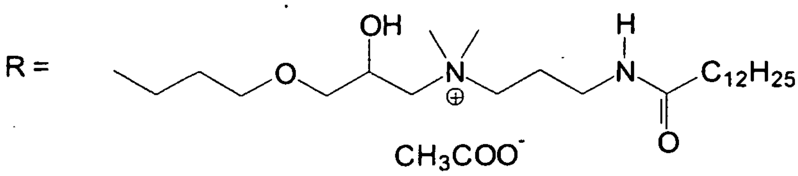

Dem Fachmann ist geläufig, dass die oben angegebene Formel eine idealisierte Strukturformel darstellt. Im Produkt liegen zusätzlich lineare und höher verzweigte Strukturen vor.

Beispiel 5:

a) Equilibrierung eines SiH-funktionellen Polysiloxans

In einem 500 mL Dreihalskolben wurden 6,7 g Methyltris(dimethylsiloxy)silan, 334 g Decamethylpentasiloxan, 0,4 g eines sauren Katalysators gemischt und 5 Stunden bei 80°C gerührt. Nach dem Abkühlen wurden 20 g $NaHCO_3$ zugegeben und bei Raumtemperatur 12 Stunden gerührt. Nach einer Filtration wurde ein klares Produkt mit einem SiH-Wert von 0,02 % erhalten.

b) Herstellung eines Epoxysiloxans

In einem 1000 mL Dreihalskolben wurden 450 g der gemäß unter 5 a) hergestellten Verbindung und 11,3 g Allylglycidylether zusammen vorgelegt und auf 100°C aufgeheizt. Anschließend wurden 15 ppm eines Platin-Katalysators zugesetzt und zwei Stunden lang gerührt. Nach einer anschließenden Reaktion erhielt man ein klares Produkt mit einem Epoxywert von 0,37 %.

c) Umsetzung zum quaternären Polysiloxan-Polymer

In einem 1000 mL Dreihalskolben wurden 31,9 g 3-N,N-Dimethyl-aminopropyl-laurylsäureamid, 8,9 g Milchsäure und 200 g Isopropanol bei Raumtemperatur gerührt. Anschließend wurden 440 g der gemäß 5 b) hergestellten Verbindung zugetropft. Anschließend wurde 8 Stunden bei 50 °C gerührt und destilliert. Es wurde eine trübe hochviskose Flüssigkeit erhalten, welches durch die folgende statistische Formel beschrieben wird:

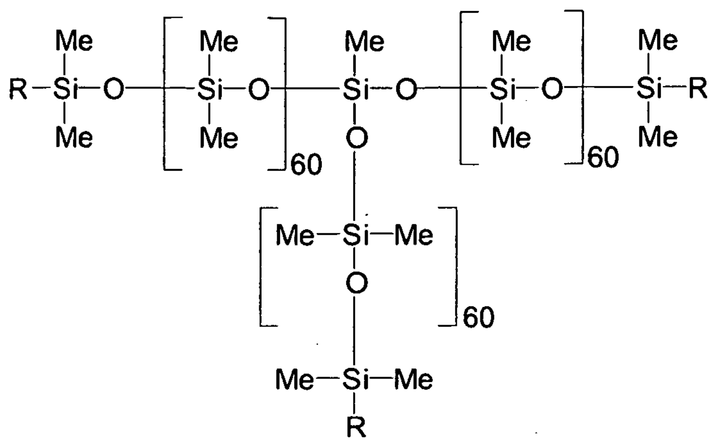

R =

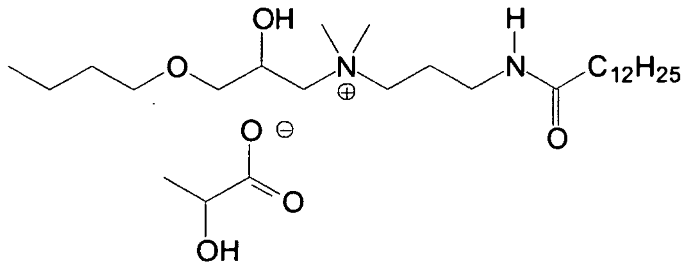

Dem Fachmann ist geläufig, dass die oben angegebene Formel eine idealisierte Strukturformel darstellt. Im Produkt liegen zusätzlich lineare und höher verzweigte Strukturen vor.

Anwendungstechnische Eigenschaften

Anwendung in der Kosmetik

Herstellung und Überprüfung von Haarbehandlungsmitteln unter Verwendung der erfindungsgemäßen Verbindungen 1, 2, 3, 4, 5.

Für die anwendungstechnische Beurteilung werden Haartressen, die für sensorische Tests verwendet werden, durch eine Dauerwellbehandlung und eine Bleichbehandlung standardisiert vorgeschädigt. Dazu werden friseurübliche Produkte verwendet. Der Testablauf, die verwendeten Basismaterialien sowie die Details der Beurteilungskriterien sind in DE 103 27 871 beschrieben.

Testrezepturen

Für die anwendungstechnische Beurteilung werden die erfindungsgemäßen Verbindungen und Vergleichsprodukte in einfachen kosmetischen Formulierungen eingesetzt.

Die Anwendungseigenschaften beim Einsatz in einem Shampoo wurden in der folgenden Rezeptur überprüft:

| Produkt | Gewichtsanteile |
|---|---|
| Natrium Laurylethersulfat (28%ig) z.B. TEXAPON NSO (Cognis) | 32 % |
| "Konditioniermittel" | 0,5 % |
| TEGO Betain F Cocamidopropyl Betaine (30%) | 10 % |

(fortgesetzt)

| Produkt | Gewichtsanteile |
|---|---|
| Kationisches Polymer zur Verbesserung der Wirksamkeit von Konditioniermitteln (cationic deposition polymer) (z.B Guar Hydroxypropyl trimonium Chloride, Polyquaternium-10) | 0,3 % |
| Wasser | ad. 100 % |
| Zitronensäure | ad. pH 6,0 ± 0,3 |

Zur Bewertung der Eigenschaften der Shampooformulierung wurde im Testablauf keine Nachbehandlung mit einer Spülung durchgeführt.

Darüber hinaus wurden die erfindungsgemäßen Produkte auch in einer einfachen Haarspülung mit folgendem Aufbau geprüft:

| Produkt | Gewichtsanteile |
|---|---|
| TEGINACID®C Ceteareth-25 | 0,5 % |
| TEGO®Alkanol 16 Cetyl Alcohol | 2,0 % |
| "Konditioniermittel" | 1,0 % |
| VARISOFT® 300 Cetrimonium Chloride (30%) | 3,3 % |
| Wasser | ad. 100 % |
| Zitronensäure | ad. pH 4,0 ± 0,3 |

Die Vorbehandlung der Haare erfolgte im Falle der Eigenschaftsprüfung von Haarspülungen durch ein Shampoo, welches keine Konditioniermittel enthält.

Als "Konditioniermittel" sind die erfindungsgemäßen Verbindungsbeispiele, Vergleichsprodukte oder Kombinationen aus erfindungsgemäßen Verbindungen und bekannten Konditioniermitteln (insbesondere Cetrimonium Chloride) bezeichnet.

Standardisierte Behandlung von vorgeschädigten Haarsträhnen mit konditionierenden Proben:

Die, wie oben beschrieben, vorgeschädigten Haarsträhnchen werden wie folgt mit dem oben beschriebenen Shampoo oder der oben beschriebenen konditionierenden Spülung behandelt: Die Haarsträhnen werden unter fließendem, warmem Wasser benetzt. Das überschüssige Wasser wird leicht von Hand ausgedrückt, dann wird das Shampoo aufgebracht und sanft im Haar eingearbeitet (1 ml/Haarsträhne (2 g)). Nach einer Verweilzeit von 1 min wird das Haar für 1 min gespült. Gegebenenfalls wird direkt im Anschluss die Spülung aufgebracht und sanft im Haar eingearbeitet (1 ml/Haarsträhne (2 g)). Nach einer Verweilzeit von 1 min wird das Haar für 1 min gespült.

Vor der sensorischen Beurteilung wird das Haar an der Luft bei 50 % Luftfeuchtigkeit und 25 °C für mindestens 12 Stunden getrocknet.

Die Zusammensetzung der Testformulierungen ist zur Vermeidung der Beeinflussung der Testergebnisse durch (normalerweise vorhandene) Formulierungsbestandteile bewusst einfach gewählt. Erfindungsgemäße Formulierungen können neben den genannten Inhaltsstoffen und/oder anstatt der genannten Inhaltsstoffe noch weitere Inhaltsstoffe enthalten. Insbesondere die Kombination mit weiteren Inhaltsstoffen kann bei den beschriebenen Effekten zu einer synergistischen Verbesserung führen. Solche Inhaltsstoffe können sein (sind aber nicht darauf beschränkt):

Tenside, Netzmittel oder Emulgatoren aus den Gruppen der anionischen, kationischen, zwitterionischen, amphoteren oder nichtionischen oberflächenaktiven Substanzen wie Fettalkoholsulfate, Fettalkoholethersulfate, Alkylsulfonate, Alkylbenzolsulfonate, Alkylsulfosuccinate, quarternäre Ammonium Salze, Alkylbetaine, Fettsäureamidoalkylbetaine, Derivatives von monomeren oder kondensierten Sacchariden wie Zuckerester, Methyl- oder Ethylglucosidfettsäureester, Alkylglucoside, ethoxylierte Fettalkohole, Fettsäurealkanolamide oder ethoxylierte Fettsäureester, Verdickungsmittel wie Kaolin, Bentonit, Fettsäuren, Fettalkohole, Stärke, Polyacrylsäure und ihre Derivate, Cellulosederivate, Guarderivate, Alginate, Chitosan, Vaselin oder Paraffin, des Weiteren Trübungsmittel, wie zum Beispiel Glycolesterderivate oder Alkohole, wie Ethanol, Propanol, Isopropanol, Propylenglykol or Glycerin, Solubilisierungemittel, Stabilisatoren, Puffersysteme, Parfümöle, Farbstoffe und insbesondere auch weitere Konditioniermittel und Pflegeadditive, wie andere kationische oder amphotere Polymere, Lanolin und seine Derivatives, Cholesterin, Ceramide, Pantothensäure, Betaine, Creatin, andere Silicone oder Siliconderivate.

Beurteilungskriterien

Die sensorischen Bewertungen erfolgen nach Noten, die auf einer Skala von 1 bis 5 vergeben werden, wobei 1 die schlechteste und 5 die beste Bewertung ist. Die einzelnen Testkriterien erhalten jeweils eine eigene Bewertung.
Die Testkriterien sind: Nasskämmbarkeit, Nassgriff, Trockenkämmbarkeit, Trockengriff, Aussehen/Glanz.
In der folgenden Tabelle werden die Ergebnisse der sensorischen Beurteilung der wie oben beschrieben durchgeführten Behandlung der Haarsträhnchen mit erfindungsgemäßen Substanzen bzw. Placebo verglichen.
In der Shampooanwendung wurde als kationisches Polymer Guar Hydroxypropyltrimonium Chloride ("Guar-Quat") verwendet

| Formulierung "einfaches Shampoo" mit | Naßkämmbarkeit | Naßgriff | Trockenkämmbarkeit | Trockengriff | Glanz |
|---|---|---|---|---|---|
| Erfindungsgemäße Verbindung 1 | 4,0 | 3,8 | 4,3 | 4,3 | 3,9 |
| Erfindungsgemäße Verbindung 2 | 3,5 | 4,0 | 4,4 | 4,3 | 4,1 |
| Erfindungsgemäße Verbindung 3 | 3,5 | 3,3 | 4,2 | 4,0 | 3,0 |
| Erfindungsgemäße Verbindung 4 | 3,0 | 3,0 | 3,5 | 3,8 | 3,0 |
| Erfindungsgemäße Verbindung 5 | 4,0 | 3,8 | 4,5 | 4,3 | 3,9 |
| Vergleichsverbindung Quaternium-80 (ABIL® Quat 3272, Goldschmidt GmbH) | 3,0 | 3,0 | 3,0 | 3,5 | 3,0 |
| Kontrolle ohne Konditioniermittel, jedoch mit "Guar-Quat" | 2,3 | 2,5 | 2,8 | 3,3 | 2,3 |
| Kontrolle Shampoo ohne Konditioniermittel | 1,5 | 1,5 | 1,3 | 2,8 | 3,5 |

Die Ergebnisse zeigen in überraschender Weise, dass die erfindungsgemäßen Verbindungen nach Beispielen 1 und 2 signifikant bessere Bewertungen erhalten als das Vergleichsprodukt "Quaternium-80". Die erfindungsgemäßen Verbindungen nach Beispielen 3 und 4 zeigen eine ähnlich gute Wirksamkeit wie Quaternium-80. Besonders deutlich ist die gute Bewertung der Glanzeigenschaften aller erfindungsgemäßen Verbindungen hervorzuheben.

| Formulierung "einfache Haarspülung" mit | Naßkämmbarkeit | Naßgriff | Trockenkämmbarkeit | Trockengriff | Glanz |
|---|---|---|---|---|---|
| Erfindungsgemäße Verbindung 1 | 4,5 | 4,0 | 4,5 | 4,5 | 4,2 |
| Erfindungsgemäße Verbindung 2 | 4,5 | 4,5 | 4,5 | 4,5 | 3,8 |
| Erfindungsgemäße Verbindung 3 | 4,0 | 4,25 | 4,5 | 4,2 | 2,8 |
| Erfindungsgemäße Verbindung 4 | 4,5 | 4,5 | 4,0 | 4,8 | 3,8 |
| Erfindungsgemäße Verbindung 5 | 4,6 | 4,0 | 4,6 | 4,5 | 4,2 |
| Kontrolle ohne erfindungsgemäße Verbindung | 4,3 | 3,5 | 4,0 | 3,8 | 2,4 |
| Kontrolle ohne erfindungsgemäße Verbindung und ohne Cetrimonium Chloride | 1,3 | 1,7 | 2,0 | 2,8 | 2,8 |

Auch in der Anwendung Haarspülung zeigen die erfindungsgemäßen Verbindungen in der sensorischen Beurteilung sehr gute kosmetische Bewertungen. Dabei wird insbesondere in den Bewertungen für Nassgriff und Trokkengriff die bereits gute Leistung des Cetrimonium Chloride durch die Kombination mit den erfindungsgemäßen Verbindungen noch weiter gesteigert. Eine signifikant bessere Bewertung wird auch beim Glanz erreicht.
Allgemein zeigt sich, dass auch das Hautgefühl an den Händen während und nach der Applikation deutlich verbessert ist, wenn erfindungsgemäße Verbindungen in der Formulierung vorhanden sind. Insbesondere in O/W Emulsionen lässt sich durch die erfindungsgemäßen Verbindungen das Hautgefühl verbessern.

So wird in der Beispielformulierung ein deutlich angenehmeres Hautgefühl durch den Einsatz der erfindungsgemäßen Verbindung erreicht.

| Phase | | Gew% |
|---|---|---|
| A | Wasser | 74,57 |
| | NaCl | 0,08 |
| B | Glycerin | 7,65 |
| | Erfindungsgemäße Verbindung 5 | 0,50 |
| C | Distearyldimonium Chloride | 4,25 |
| | Petrolatum | 4,55 |
| | Isopropyl Palmitate | 4,25 |
| | Cetyl Alcohol | 3,75 |
| | Dimethicone | 0,40 |
| D | Konservierung | q.s. |
| | Citronensäure | ad. pH 4,2 bis 4,3 |
| Herstellung:<br>Phase A mischen und auf 70°C erwärmen.<br>Phase B homogen vermischen und zu Phase A einrühren, bei 70°C halten.<br>Die Inhaltsstoffe der Phase C nacheinander zur Kombination von Phase A und B geben und bei jeder Zugabe jeweils homogen verrühren. Dabei die Temperatur bei 70°C halten.<br>Danach die vollständige Mischung für ca. 2 Minuten homogenisieren. Unter Rühren abkühlen und ggfs. Konservierungsmittel zusetzen. | | |

Ein weiterer Gegenstand der Erfindung ist daher die Verwendung der erfindungsgemäßen Verbindungen in Formulierungen zur Reinigung und Pflege der Haut wie beispielsweise Duschprodukten, Badeprodukten, Flüssigseifen.

Anwendung in der Autopflege

Weiterhin ist Gegenstand dieser Erfindung der Einsatz erfindungsgemäßer Verbindungen in der gewerblichen Autowäsche in Trocknungshilfsmitteln in der Autowaschstraße. Hier wird nach dem mit anionischen Tensiden durchgeführten Reinigungsschritt eine Behandlung mit einem kationischen Mittel nachgeschaltet, die für Glanz und Hydrophobie auf dem Autolack sorgt und gleichzeitig die anschließende Trocknung des Fahrzeugs durch ein Gebläse ermöglicht. Die Einwirkzeit dieses Mittels ist bei Autowaschanlagen von besonderer Bedeutung, um den Durchsatz an Fahrzeugen und damit die Effizienz zu erhöhen. Der Einsatz erfindungsgemäßer Verbindungen wurde in praxisgerechten Formulierungen überprüft, wobei überraschenderweise eine erhebliche Verkürzung der Aufrisszeit beobachtet wurde, auch im Vergleich zu literaturbekannten quarternären Siliconverbindungen.

Folgende Basisrezeptur wurde getestet:

| | |
|---|---|
| Butyldiglycol | 8,5 % |
| Dipropylenglycol-butylether | 5,5 % |
| 9-Octadecensaure (Z)-, Reaktionsprodukte mit Triethanolamin, Dimethylsulfat-quaternisiert | 12,0 % |
| Octylpalmitat | 5,0 % |
| Essigsäure | 0,5 % |
| Wasser ad 100 | |
| | |

Formulierung 8

Die Basisrezeptur ist Formulierung Nr. 8.

Formulierung 9

Die Basisrezeptur mit 0,8 % Wirkstoff quaternärer Siliconverbindung gemäß Patent EP 0 294 643 ist Formulierung 9.

Formulierung 10

Die Basisrezeptur mit 0,8 % Wirkstoff quaternärer Siliconverbindung gemäß dieser Schrift mit N = 50 ist Formulierung 10.

Formulierung 11

Die Basisrezeptur mit 0,8 % Wirkstoff quaternärer Siliconverbindung gemäß dieser Schrift mit N = 80 ist Formulierung 11.

Diese Formulierungen wurden praxisgerecht mit Leitungswasser 1 : 1000 verdünnt und die Verdünnungen im Aufrissverhalten untersucht.

Kennzeichnend für die Leistungsfähigkeit ist der Aufriss des Wasserfilms auf dem Autolack wie auch den Glasflächen des Fahrzeuges nach der Aufgabe des Trocknungshilfsmittels. Während eine Bestimmung des Aufrisses auf lackierten Oberflächen schwierig zu reproduzieren ist, sind Glasoberflächen hierzu sehr geeignet.

Das Aufrissverhalten wurde folgendermaßen bestimmt:
Es wird die Zeit gemessen, die notwendig ist, um einen definierten Wasserfilm auf Glas zu durchdringen und das Glas zu entnetzen. Es wird die erste Reaktionszeit sowie das vollständige Verdrängen des Wassers von einem Objektträger festgehalten.

Spiegelfliese
Objektträger 76x26 mm (3x1 inch)
Pipette 3 ml Kunststoff
Dosierpipette 100 µl
Wasser definierter Qualität; Angabe der Leitfähigkeit
Stoppuhr
Bunsenbrenner

Proben werden gemäß Angabe in Wasser verdünnt vermessen, zumeist in einer 1 : 1000 Verdünnung. Der Objektträger wird entstaubt und über einer Flamme kurz abgeflammt, um eine absolut saubere rückstandsfreie Oberfläche zu gewährleisten.

0,5 ml Wasser werden mit Hilfe einer Pipette als gleichmäßiger Film auf dem Objektträger aufgetragen. Sollte sich kein Film bilden lassen, muss der Objektträger nochmals gereinigt oder verworfen werden. Anschließend werden 50 µl der Gebrauchsverdünnung der Trocknungshilfe mittig auf die Wasseroberfläche appliziert und die Stoppuhr gestartet. Es werden nun der Beginn der Entnetzung und der Durchbruch des sich zurückziehenden Wassers auf der 26 mm Seite festgehalten. Hierdurch lässt sich die Reaktionszeit und die Aufrissgeschwindigkeit festhalten.
Die Angabe erfolgt in Sekunden.

Die Tabelle zeigt folgende Ergebnisse:

| | Beginn des Aufrisses | Ende des Aufrisses |
|---|---|---|
| Verdünnung aus Formulierung 8 | 10 s | 30 s |
| Verdünnung aus Formulierung 9 | 15 s | 30 s |
| Verdünnung aus Formulierung 10 | 3 s | 15 s |
| Verdünnung aus Formulierung 11 | 7 s | 25 s |

Es zeigt sich, dass insbesondere der Aufriss mit der erfindungsgemäßen Verbindung mit N = 50 einen erheblichen Effekt aufweist.

Als Skalierung kann folgende Tabelle verwendet werden:

| Beginn des Aufrisses | | | Ende des Aufrisses | | |
|---|---|---|---|---|---|
| optimal | <10 s | | gut | < 10 s | |
| gut | 11 - 20 s | | optimal | 11 - 20 s | |
| mäßig | 21 - 45 s | | gut | 21 - 30 s | |
| schlecht | > 45 s | | mäßig | 31 - 45 s | |
| | | | schlecht | < 45 s | |

Der Aufriss soll nicht zu schnell enden, da sonst schwer entfernbare Mikrotropfen entstehen, die nach dem Trocknen Spuren hinterlassen.

Gleichzeitig wirken sich die Verbindungen sichtbar positiv auf den Glanz des getrockneten Fahrzeugs aus.

## Patentansprüche

1. Polysiloxane der allgemeinen Formel I:

$$[M'\ D_n]_3T \qquad \text{Formel I}$$

worin die Substituenten und Indices bedeuten:

$M' = XSiY_2O_{1/2}$
$D = SiY_2O_{2/2}$
$T = SiZO_{3/2}$
X = gleiche oder verschiedene Ammoniumfunktionen tragende organische Reste,
Y = gleiche oder verschiedene Reste aus der Gruppe Alkyl, Aryl, oder Alkaryl mit 1 bis 30 C-Atomen,
Z = gleiche oder verschiedene Reste aus der Gruppe Alkyl, Aryl, oder Alkaryl mit 1 bis 30 C-Atomen,
n = 2 bis 200.

2. Polysiloxane gemäß Anspruch 1, **dadurch gekennzeichnet, dass**

X = -R1-R2 ist, mit der Bedeutung
R1 gleiche oder verschiedene zweiwertige Reste, ausgewählt aus der Gruppe

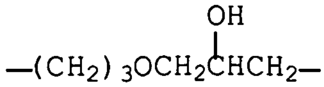

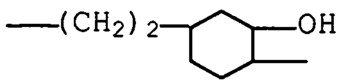

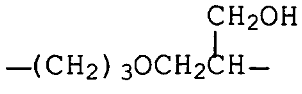

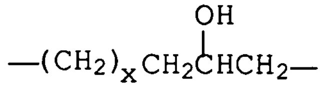

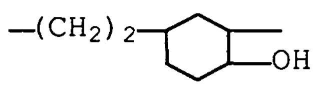

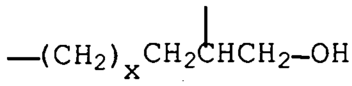

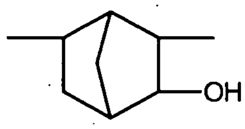

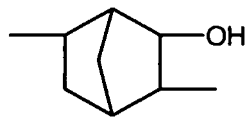

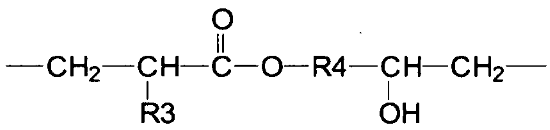

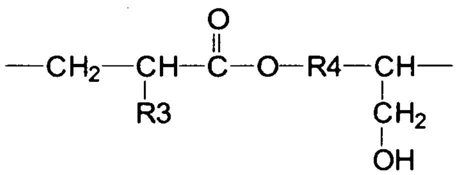

R2 ausgewählt ist aus der Gruppe bestehend aus

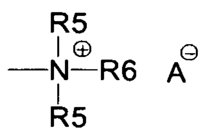

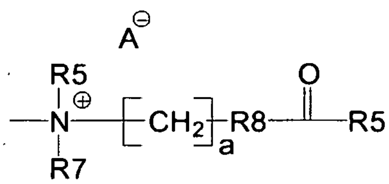

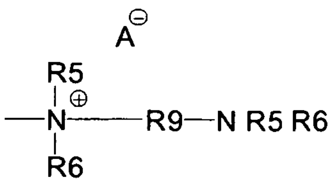

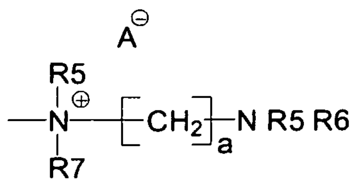

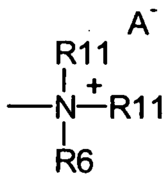

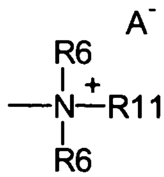

R3 sind gleiche oder verschiedene Reste aus der Gruppe Wasserstoff oder Alkyl mit 1 bis 6 C-Atomen,
R4 sind gleiche oder verschiedene zweiwertige Kohlenwasserstoffreste, die gegebenenfalls Etherfunktionen

enthalten,
R5, R6, R7 sind jeweils unabhängig voneinander Wasserstoff oder Alkylreste mit 1 bis 30 C-Atomen,
R8 sind gleiche oder verschiedene Reste aus der Gruppe -O-; -NR10,
R9 sind gleiche oder verschiedene gegebenenfalls verzweigte divalente Kohlenwasserstoffreste,
R10 sind gleiche oder verschiedene Reste aus der Gruppe Wasserstoff oder Alkyl mit 1 bis 6 C-Atomen,
R11 sind gleiche oder verschiedene Reste der allgemeinen Formel:

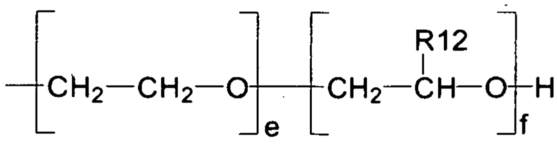

R12 sind gleiche oder verschiedene Alkyl-, Aryl- oder Alkarylreste mit 1 bis 30 C-Atomen, die gegebenenfalls Etherfunktionen enthalten,
e 0 bis 20,
f 0 bis 20,
e + f >= 1,
x 2 bis 18,
a 2 bis 18,
$A^-$ sind gleiche oder verschiedene Gegenionen zu den positiven Ladungen an den quaternierten Stickstoffgruppen, ausgewählt aus anorganischen oder organischen Anionen der Säuren HA, sowie deren Derivate.

**3.** Polysiloxane gemäß Anspruch 2, **dadurch gekennzeichnet, dass**

R1 die Bedeutung

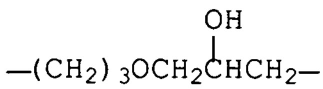

hat.

**4.** Verfahren zur Herstellung von Polysiloxanen gemäß einem der Ansprüche 1 bis 3, **dadurch gekennzeichnet, dass** zunächst Aryltris(dimethylsiloxy)silane und/oder Alkyltris(dimethylsiloxy)silane mit Siloxancyclen equilibriert werden, anschließend Doppelbindungen enthaltende Epoxyde mit dem entstandenen SiH-funktionellen Equlibrat hydrosilyliert werden und die so erhaltenen Epoxysiloxane schließlich mit tertiären Aminen zu den entsprechenden quaternären Siloxanen umgesetzt werden.

**5.** Verwendung der Verbindungen gemäß einem der Ansprüche 1 bis 3 als Additiv in gegebenenfalls tensidhaltigen wässrigen Pflege- und Reinigungsformulierungen.

**6.** Verwendung der Verbindungen gemäß einem der Ansprüche 1 bis 3 als Konditioniermittel für Haarbehandlungsmittel, Haarnachbehandlungsmittel und zur Verbesserung der Haarstruktur.

**7.** Verwendung der Verbindungen gemäß einem der Ansprüche 1 bis 3 zur Herstellung von glanzverbessernden Pflegeformulierungen.

**8.** Verwendung der Verbindungen gemäß einem der Ansprüche 1 bis 3 zur Herstellung von Formulierungen zur Reinigung der Haut.

**9.** Verwendung der Verbindungen gemäß einem der Ansprüche 1 bis 3 zur Herstellung von Formulierungen zur Pflege der Haut.

**10.** Verwendung der Verbindungen gemäß einem der Ansprüche 1 bis 3 zur Herstellung von Formulierungen zur Reinigung und Pflege von harten Oberflächen.

**11.** Verwendung der Verbindungen gemäß einem der Ansprüche 1 bis 3 zur Herstellung von Formulierungen zur Reinigung und Pflege von Fahrzeugen.

**12.** Verwendung der Verbindungen gemäß einem der Ansprüche 1 bis 3 als Additiv in Trocknungshilfen für Autowaschstraßen.

## Claims

**1.** Polysiloxanes of the general formula I:

$$[M' D_n]_3 T \qquad \text{formula I}$$

in which the substituents and indices are:

M' = $XSiY_2O_{1/2}$
D = $SiY2O_{2/2}$
T = $SiZO_{3/2}$
X = identical or different organic radicals carrying ammonium functions,
Y = identical or different radicals from the group consisting of alkyl, aryl or alkaryl having 1 to 30 carbon atoms,
Z = identical or different radicals from the group consisting of alkyl, aryl or alkaryl having 1 to 30 carbon atoms,
n = from 2 to 200.

**2.** Polysiloxanes according to Claim 1, **characterized in that**

X = -R1-R2, where
R1 are identical or different divalent radicals selected from the group consisting of

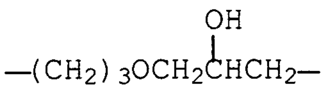

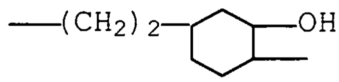

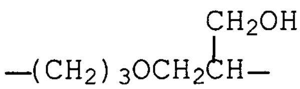

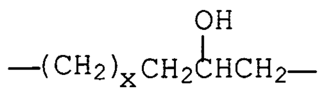

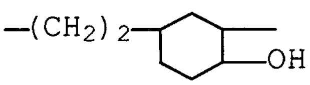

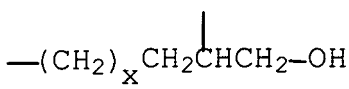

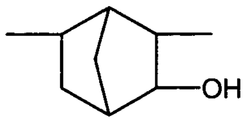

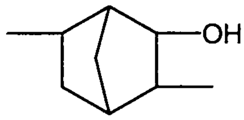

.

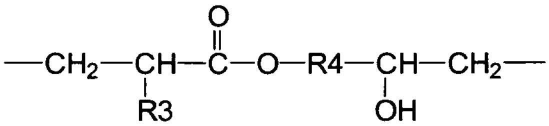

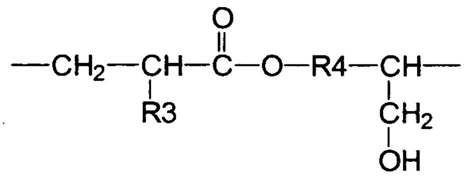

R2 is selected from the group consisting of

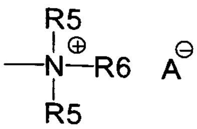

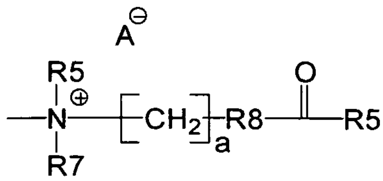

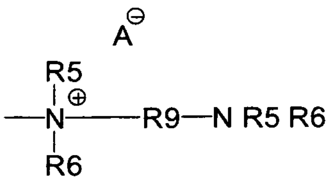

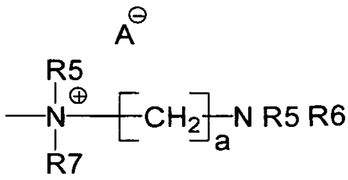

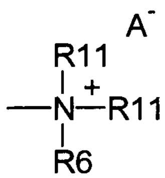

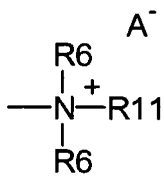

R3 are identical or different radicals from the group consisting of hydrogen or alkyl having 1 to 6 carbon atoms,
R4 are identical or different divalent hydrocarbon radicals which optionally contain ether functions,
R5, R6, R7 are, in each case independently of one another, hydrogen or alkyl radicals having 1 to 30 carbon atoms,
R8 are identical or different radicals from the group -O-; -NR10,
R9 are identical or different optionally branched divalent hydrocarbon radicals,
R10 are identical or different radicals from the group consisting of hydrogen or alkyl having 1 to 6 carbon atoms,
R11 are identical or different radicals of the general formula:

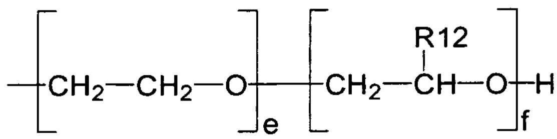

R12 are identical or different alkyl, aryl or alkaryl radicals having 1 to 30 carbon atoms which optionally contain ether functions,
e is from 0 to 20,
f is from 0 to 20,
e + f > = 1,
x is from 2 to 18,
a is from 2 to 18,
$A^-$ are identical or different counter ions to the positive charges on the quaternized nitrogen groups, selected from inorganic or organic anions of the acids HA, and derivatives thereof.

**3.** Polysiloxanes according to Claim 2, **characterized in that**

R1 has the meaning

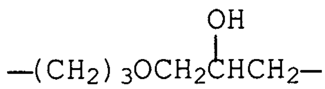

**4.** Process for the preparation of polysiloxanes according to any of Claims 1 to 3, **characterized in that** first aryltris (dimethylsilyloxy)silanes and/or alkyltris-(dimethylsilyloxy)silanes are equilibrated with cyclic siloxane compounds, epoxides containing double bonds are then hydrosilylated with the resulting SiH-functional equilibration product, and the epoxy siloxanes thus obtained are finally reacted with tertiary amines to give the corresponding quaternary siloxanes.

**5.** Use of the compounds according to any of Claims 1 to 3 as an additive in optionally surfactant-containing aqueous care and cleaning formulations.

**6.** Use of the compounds according to any of Claims 1 to 3 as conditioners for hair treatment compositions, and hair aftertreatment compositions and for improving the hair structure.

**7.** Use of the compounds according to any of Claims 1 to 3 for the preparation of gloss-improving care formulations.

**8.** Use of the compounds according to any of Claims 1 to 3 for the preparation of formulations for cleansing the skin.

**9.** Use of the compounds according to any of Claims 1 to 3 for the preparation of formulations for the care of the skin.

**10.** Use of the compounds according to any of Claims 1 to 3 for the preparation of formulations for the cleaning and care of hard surfaces.

**11.** Use of the compounds according to any of Claims 1 to 3 for the preparation of formulations for the cleaning and care of vehicles.

**12.** Use of the compounds according to any of Claims 1 to 3 as an additive in drying aids for car washing installations.

## Revendications

**1.** Polysiloxanes de formule générale I :

$$[M' D_n]_3 T \qquad \text{formula I}$$

dans laquelle les substituants et indices ont les significations suivantes :

$M' = XSiY_2O_{1/2}$
$D = SiY_2O_{2/2}$
$T = SiO_{3/2}$
X représente des radicaux organiques identiques ou différents, portant des fonctions ammonium,
Y représente des radicaux identiques ou différents, choisis dans l'ensemble constitué par les groupes alkyle, aryle et alkaryle ayant de 1 à 30 atomes de carbone,
Z représente des radicaux identiques ou différents, choisis dans l'ensemble constitué par les groupes alkyle, aryle et alkaryle ayant de 1 à 30 atomes de carbone,
n = 2 à 200.

**2.** Polysiloxanes selon la revendication 1,
**caractérisés en ce que**

X = -R1-R2, où
R1 représente des radicaux divalents identiques ou différents, choisis dans l'ensemble constitué par

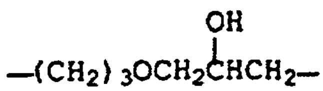
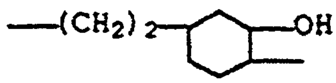

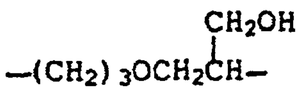
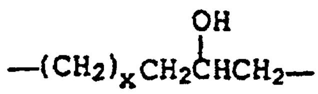

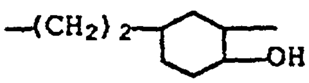
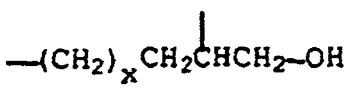

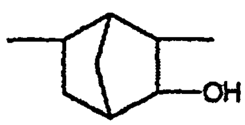
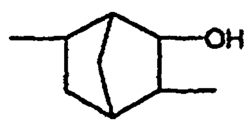

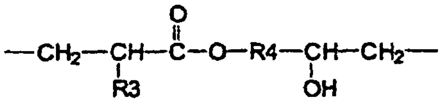

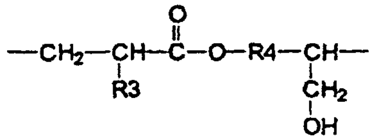

R2 est choisi dans l'ensemble constitué par

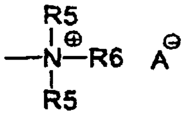

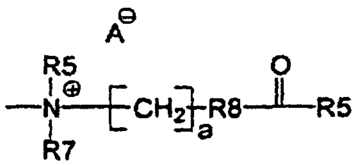

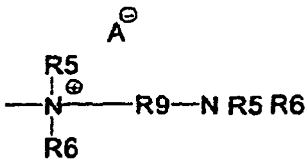

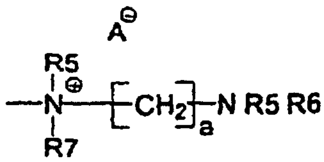

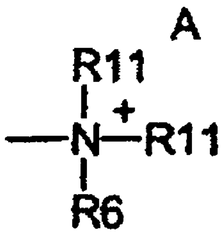

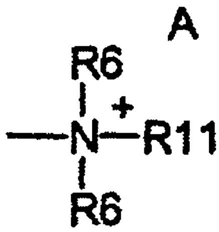

R3 sont des radicaux identiques ou différents, choisis dans l'ensemble constitué par l'atome d'hydrogène et les groupes alkyle ayant de 1 à 6 atomes de carbone,
R4 sont des radicaux hydrocarbonés divalents identiques ou différents, qui éventuellement contiennent des fonctions éther,
R5, R6, R7 représentent, chacun indépendamment, un atome d'hydrogène ou des radicaux alkyle ayant de 1 à 30 atomes de carbone,
R8 sont des radicaux identiques ou différents, choisis parmi -O- et -NR10,
R9 sont des radicaux hydrocarbonés divalents identiques ou différents, éventuellement ramifiés,
R10 sont des radicaux identiques ou différents, choisis dans l'ensemble constitué par l'atome d'hydrogène et des groupes alkyle ayant de 1 à 6 atomes de carbone,
R11 sont des radicaux identiques ou différents, de formule générale :

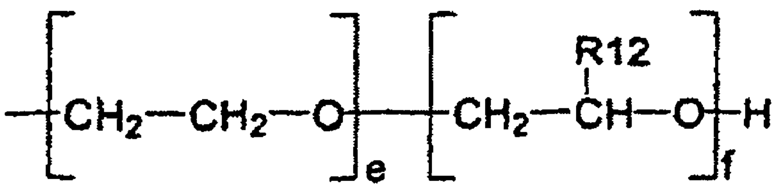

R12 sont des radicaux alkyle, aryle ou alkaryle identiques ou différents, ayant de 1 à 30 atomes de carbone, qui contiennent éventuellement des fonctions éther,
e va de 0 à 20,
f va de 0 à 20,
e + f est > 1,
x va de 2 à 18,
a va de 2 à 18,
$A^-$ sont des ions identiques ou différents, opposés aux charges positives sur les groupes azotés rendus quaternaires, choisis parmi des anions organiques ou inorganiques des acides HA, ainsi que de leurs dérivés.

**3.** Polysiloxanes selon la revendication 2,
**caractérisés en ce que**

R1 représente

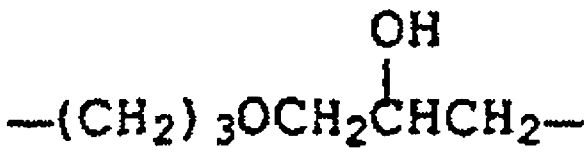

**4.** Procédé pour la préparation de polysiloxanes selon l'une quelconque des revendications 1 à 3,
**caractérisé en ce que** d'abord on équilibre des aryltris(diméthylsiloxy)silanes et/ou des alkyltris(diméthylsiloxy) silanes avec des cycles siloxane, puis on soumet des époxydes contenant des doubles liaisons avec le produit d'équilibrage à fonction SiH résultant et enfin on fait réagir les époxysilanes ainsi obtenus avec des amines tertiaires, pour obtenir des siloxanes quaternaires.

**5.** Utilisation des composés selon l'une quelconque des revendications 1 à 3, en tant qu'additif dans des compositions aqueuses de soin et de nettoyage contenant éventuellement des tensioactifs.

**6.** Utilisation des composés selon l'une quelconque des revendications 1 à 3, en tant que conditionneur pour des produits de traitement capillaire, des produits d'après-traitement capillaire et destiné à l'amélioration de la structure des cheveux.

**7.** Utilisation des composés selon l'une quelconque des revendications 1 à 3, pour la fabrication de compositions de soin améliorant le brillant.

**8.** Utilisation des composés selon l'une quelconque des revendications 1 à 3, pour la fabrication de compositions destinées au nettoyage de la peau.

**9.** Utilisation des composés selon l'une quelconque des revendications 1 à 3, pour la fabrication de compositions destinées au soin de la peau.

**10.** Utilisation des composés selon l'une quelconque des revendications 1 à 3, pour la fabrication de compositions destinées au nettoyage et à l'entretien de surfaces dures.

11. Utilisation des composés selon l'une quelconque des revendications 1 à 3, pour la fabrication de compositions destinées au nettoyage et à l'entretien de véhicules.

12. Utilisation des composés selon l'une quelconque des revendications 1 à 3, en tant qu'additif dans des produits auxiliaires de séchage pour des stations de lavage d'automobiles.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 1493384 B
- DE 3719086
- EP 0282720 A
- DE 3340708 A
- DE 10107772
- EP 1439200 A
- DE 10327871
- EP 0294643 A


### In der Beschreibung aufgeführte Nicht-Patentliteratur


- **K. F. DE POLO.** A Short Textbook of Cosmetology. Verlag für chemische Industrie, 2000
- Special Purpose Cleaning Formulations: Auto Care. **F. MÜLLER ; J. PEGGAU ; S. ARIF.** Handbook of Detergents, Part D: Formulation. CRC Press, 2006, 261-278